# EUROPEAN PATENT SPECIFICATION

(11) **EP 3 576 771 B1**
(45) Date of publication and mention of the grant of the patent: **09.03.2022**
(21) Application number: 18747581.9
(22) Date of filing: 31.01.2018
(51) Int. Cl.: A61K 38/09, A61P 35/00, A61K 9/00, A61K 9/06, A61K 9/16, A61K 47/34

(54) **COMPOSITIONS AND METHODS FOR LONG TERM RELEASE OF GANADOTROPIN-RELEASING HORMONE (GNRH) ANTAGONISTS**
ZUSAMMENSETZUNGEN UND VERFAHREN ZUR LANGZEITFREISETZUNG VON ANTAGONISTEN DES GONADOTROPINFREISETZENDEN HORMONS (GNRH)
COMPOSITIONS ET MÉTHODES POUR LA LIBÉRATION À LONG TERME D'ANTAGONISTES DE L'HORMONE DE LIBÉRATION DES GONADOTROPINES (GNRH)

(30) Priority: 31.01.2017 US 201762452788 P
(43) Date of publication of application: 11.12.2019
(60) Divisional application: 21218063.2
(73) Proprietor: Veru Inc., Miami, Florida 33137 (US)
(72) Inventor: RAVI, Kacker, Lexington, Massachusetts 02421 (US); MITCHELL S., Steiner, Germantown, Tennessee 38139 (US)
(74) Representative: Kutzenberger Wolff & Partner
(86) International application number: PCT/US2018/016241
(87) International publication number: WO 2018/144603

(56) References cited:
- CN-A- 102 145 160
- GB-A- 2 228 262
- US-A- 5 744 153
- US-A1- 2003 133 964
- US-A1- 2005 042 294
- US-A1- 2006 003 008
- US-A1- 2007 003 592
- US-A1- 2007 196 416
- US-A1- 2011 200 679
- US-A1- 2012 202 743
- US-A1- 2013 203 796
- US-A1- 2016 106 804
- Www Posterpresentations ET AL: "RESEARCH POSTER PRESENTATION DESIGN 2019", , 1 February 2020 (2020-02-01), XP055697671, Retrieved from the Internet: URL:https://verupharma.com/wp-content/uplo ads/2020/02/VERU-100-Poster-02-2020.pdf [retrieved on 2020-05-25] & Anonymous: "Determination of the ability of a novel, long-acting subcutaneous GnRH antagonist, VERU-100, to castrate without a testosterone surge in a rat model. | Journal of Clinical Oncology", , 19 February 2020 (2020-02-19), XP055697672, Retrieved from the Internet: URL:https://ascopubs.org/doi/10.1200/JCO.2 020.38.6_suppl.128 [retrieved on 2020-05-25]
- SCHWACH G ET AL: "Biodegradable microparticles for sustained release of a new GnRH antagonist - part I: screening commercial PLGA and formulation technologies", EUROPEAN JOURNAL OF PHARMACEUTICS AND BIOPHARMACEUTICS, ELSEVIER SCIENCE PUBLISHERS B.V., AMSTERDAM, NL, vol. 56, no. 3, 1 November 2003 (2003-11-01), pages 327-336, XP004470468, ISSN: 0939-6411, DOI: 10.1016/S0939-6411(03)00096-1

## Description

### FIELD OF THE INVENTION

The invention relates to compositions and methods for long term release of Gonadotropin-releasing hormone (GnRH) antagonists. Specifically, the invention relates to polymer based compositions for controlled release of GnRH antagonists. In the following, references to methods of treatment in the subsequent paragraphs of this description are to be interpreted as references to the compounds, pharmaceutical compositions and medicaments for use in a method of treatment of the human (or animal) body by therapy (or for diagnosis).

### BACKGROUND OF THE INVENTION

The hypothalamic hormone, gonadotropin-releasing hormone (GnRH) (also known as luteinizing hormone releasing hormone (LHRH)), controls the secretion of the gonadotropins, luteinizing hormone (LH) and follicle stimulating hormone (FSH) from the anterior pituitary gland. GnRH is secreted by the hypothalamus, and stimulates secretion of luteinizing hormone (LH) and follicle stimulating hormone (FSH). Analogues of GnRH are currently used to treat many medical conditions that require manipulation of the production of the sex hormones, testosterone and estrogen. Schally et al. (*Schally 1971*) isolated, identified the amino acid sequence, and synthesized the peptide hormone GnRH. Deletion or replacement of different amino acids of GnRH peptide has resulted in the discovery of GnRH agonist analogues that demonstrate greater potency for the secretion of LH and FSH. A paradoxical clinical effect occurs when agonistic analogues are used continuously such that after the chronic, and relatively long period (2-3 weeks) of stimulation of the secretion of LH and FSH, there is actually an inhibition of LH and FSH release and consequent suppression of sex steroid production. (*Reissmann 2000*)*.* In certain medical conditions, however, an immediate and dose-dependent suppression of LH and FSH is desired. Over 20 years ago, Schally and Revier synthesized the 1^{st} generation analogues of GnRH antagonist analogues which were too lipophilic and induced histamine release. (*Schmidt 1984; Hahn 1985*)*.* The 2^{nd} generation GnRH antagonist analogues were made by incorporating further amino acid substitutions (*Bajusz 1988; Rivier 1993*) that resulted in potentially safer and more effective decapeptide analogues. Examples of newer generation GnRH antagonist analogues include abarelix, degarelix, ganirelix, ozarelix, cetrorelix, taverelix, antarelix, and iturelix.

Clinical development and medical applications of these GnRH antagonist analogues have been either successful or attempted for controlled ovarian stimulation for assisted reproductive techniques, uterine myoma, ovarian cancer, benign prostatic hyperplasia, and prostate cancer. In certain diseases and conditions, the major limitation for successful application of the GnRH antagonist analogue has been having only a short acting formulation where longer acting depot formulations would be more advantageous.

Accordingly, there exists a need for very long acting controlled or extended release formulations of a GnRH antagonist (also called a LHRH antagonist).

### SUMMARY OF THE INVENTION

In one aspect, the invention relates to a composition comprising cetrorelix and a polymer, said polymer comprising a poly-lactic co-glycolic acid (PLGA) in a lactide: glycolide molar ratio between 50:50 and 100:0, wherein cetrorelix is present in an amount of 5% - 90% of the mass of said composition, and said polymer is present in an amount of 10% - 50% of the mass of said composition, and wherein said composition is capable of extending the release of cetrorelix in a subject for a period ranging from 1 month to 6 months.

In an exemplary embodiment, the composition is capable of achieving a therapeutic effect within, for example, 24 hrs and maintains therapeutic effect for at least 90 days for >95% percent of treated patients. In a particular embodiment, the composition is in the form of a hydrogel. In another particular embodiment, the composition is in the form of microspheres.

The composition of the invention can administered using a suitable method. In one aspect, the composition of the invention is an injectable composition, which is administered with one injection or two injections administered at the same time using, for example, a 2 1 gauge needle or smaller, with a total injection volume, for example, less than 4 mL. Injections may be subcutaneous or intramuscular.

In another aspect, the invention relates to a method for extending the release of cetrorelix in a subject for a period ranging from 1 month to 6 months, the method comprising administering to said subject the claimed composition comprising cetrorelix and a polymer, said polymer comprising poly-lactic co-glycolic acid (PLGA) in a lactide:glycolide molar ratio between 50:50 and 100:0, wherein cetrorelix is present in an amount of 5%-90% of the mass of said composition, and said polymer is present in an amount of 10%-50% of the mass of said composition.

In another aspect, the invention relates to a method of maintaining a therapeutic level of cetrorelix in a subject for a period ranging from 1 month to 6 months, the method comprising administering to said subject the claimed composition comprising cetrorelix and a polymer, said polymer comprising poly-lactic co-glycolic acid (PLGA) in a lactide:glycolide molar ratio between 50:50 and 100:0, wherein cetrorelix is present in an amount of 5%-90% of the mass of said composition, and said polymer is present in an amount of 10%-50% of the mass of said composition.

In another aspect, the composition of the invention allows for consistent release of the active agent from the drug delivery vehicle with no more than 25% variation plus an encapsulation efficiency of over 70%. In yet another aspect, the composition of the invention allows releasing the active agent from the drug delivery vehicle with >85% intact over the entire duration of release.

Other features and advantages of the present invention will become apparent from the following detailed description examples and figures.

### BRIEF DESCRIPTION OF THE DRAWINGS

**Figure 1** shows *in vitro* release of cetrorelix (CRX) from microspheres composed of different polymers and carriers.
**Figure 2** shows *in vitro* release of cetrorelix from microspheres composed of different polymers loaded with cetrorelix in 35% acetic acid/65% H₂O carrier.
**Figure 3** shows daily levels of cetrorelix release from microspheres composed of different polymers.
**Figure 4A** shows the morphology of cetrorelix coated 10CP10C20-D23 beads (12.7% cetrorelix in 65% acetic acid (HAc):25% water).
**Figure 4B** shows the morphology of cetrorelix coated 20CP15C50-D23 beads comprising 13.4% cetrorelix).
**Figure 5** shows cetrorelix plasma concentration following administration of cetrorelix-loaded PLGA microspheres to rats.
**Figure 6A** shows long term cetrorelix plasma concentration following administration of microspheres loaded with cetrorelix and salt formulations, to rats.
**Figure 6B** shows plasma concentration following administration of microspheres loaded with cetrorelix and salt formulations over first 24 hours following administration (*i.e.* burst).
**Figure 7A** shows comparative cetrorelix plasma concentrations for microspheres loaded with cetrorelix with and without salt.
**Figure 7B** shows comparative cetrorelix plasma concentrations for microspheres loaded with cetrorelix with and without salt over first 24 hours following administration.
**Figure 8** shows shows comparative cetrorelix plasma concentrations for microspheres loaded with cetrorelix with and without salt after dose normalization.
**Figure 9A** shows rat serum testosterone levels following administration of various cetrorelix microspheres formulations.
**Figure 9B** shows rat serum testosterone levels following administration of various cetrorelix microspheres formulations over first 24 hours following administration (*i.e.* burst).
**Figure 10** shows cumulative cetrorelix *in vitro* release from PLGA microspheres.
**Figure 11** shows cumulative cetrorelix *in vitro* release from RG502H/RG752H (30% PLGA)-salt microsphere formulations.
**Figure 12** shows cumulative cetrorelix *in vitro* release from RG752H (40% PLGA) salt microsphere formulations.
**Figure 13** shows cumulative cetrorelix *in vitro* release from RG502H/RG752H (40% PLGA)-salt microsphere formulations.

### DETAILED DESCRIPTION OF THE INVENTION

The invention relates to a controlled release composition comprising a gonadotropin-releasing hormone (GnRH) antagonist in combination with one or more polymers and/or salts.

The GnRH antagonist is cetrorelix.

In one aspect, provided herein is a composition, said composition comprising: a therapeutically effective amount of a GnRH antagonist (i.e. cetrorelix) in combination with a polymer, wherein said polymer is poly-lactic co-glycolic acid (PLGA), wherein said composition is capable of releasing said GnRH antagonist for a long term (e.g., more than 90 days).

The PLGA polymers in the compositions of the present invention have lactide:glycolide weight ratio ranging from 50:50 to 100:0. In particular embodiments, the lactide to glycolide ratio is about, 55:45, 60:40, 65:35, 70:30, 75:25, 80:20, 85:15, 90:10, or 95:5.

In a further aspect, the PLGA polymers the compositions of the present invention may comprise a mixture of two or more PLGA polymers each having a different glycolide and lactide fractions. For example, the mixture may include a first PLGA polymer having equal amount of glycolide and lactide (RG502H) and a second PLGA polymer having 25% glycolide and 75% lactide (RG752H). The proportions of the first PLGA polymer and the second PLGA polymer may vary, for example the ratio of RG502H to RG752H can range from about 100:0 to about 0:100.

In one aspect, the composition is a flowable composition capable of forming an *in situ* implant in a subject. In one example, the composition includes a biodegradable thermoplastic polymer (i.e. PLGA), a biocompatible solvent; and a GnRH antagonist (i.e. cetrorelix).

The biodegradable thermoplastic polymer can be substantially insoluble in aqueous medium or body fluid. Biodegradable thermoplastic polymers are well known in the art and fully described in U.S. Patents 6,565,874; 5,324,519; 4,938,763; 5,702,716; 5,744,153; and 5,990,194

The type, amount, and molecular weight, of biodegradable thermoplastic polymer present in the composition may depend upon one or more desired properties of the controlled release implant.

Examples of types of biodegradable thermoplastic polyesters are well known in the art and fully described in U.S. Patent 6,565,874. In a particular embodiment, the suitable biodegradable thermoplastic polyester is 50:50 poly (DL-lactide-co-glycolide) having a carboxy terminal group or is 75:25 poly(DL-lactide-co-glycolide) with a carboxy terminal group that is protected. Other suitable copolymers, known to one of skilled in the art, can also be used.

The amount of biodegradable thermoplastic polymer, in the composition, can be any suitable amount, known to one of skilled in the art. The amount, in the composition, may range from about 30 wt. % to 50 wt. %; or from about 35 wt. % to about 45 wt. %. In a particular embodiment, the amount is 10, 20, 25, 30, 35, 40, 45, or 50 wt. %.

The molecular weight of biodegradable thermoplastic polymer, in the composition, can be any suitable molecular weight, known to one of skilled in the art. The molecular weight may range from about 10,000 to about 50,000; from about 15,000 to about 45,000; from about 20,000 to about 40,000; or from about 20,000 to about 30,000. In a particular embodiment, the molecular weight is approximately 10,000, 15,000, 20,000, 25,000, 30,000, 35,000, 40,000, 45,000, or 50,000.

Preferably, the biodegradable thermoplastic polyester has an average molecular weight ranging from about 23,000 to about 45,000 or from about 15,000 to about 24,000.

The biocompatible solvent can be a biocompatible polar aprotic solvent. In one embodiment, the solvent is miscible to dispersible in aqueous medium or body fluid. Suitable polar aprotic solvents are well known in the art and fully described in, for example., in Aldrich Handbook of Fine Chemicals and Laboratory Equipment, Milwaukee, Wis. (2000) and U.S. Patents 6,565,875, 5,324,519; 4,938,763; 5,702,716; 5,744,153; and 5,990,194.

In one aspect, the solvent of the invention is capable of diffusing into body fluid so that the flowable composition coagulates or solidifies. In another aspect, the solvent of the invention is biodegradable. In yet another aspect, the solvent of the invention is non-toxic. As set forth in U.S. Patent 6,565,875, examples of suitable polar aprotic solvents include polar aprotic solvents having an amide group, an ester group, a carbonate group, a ketone, an ether, a sulfonyl group, or a combination thereof.

In one embodiment, the polar aprotic solvent is *N*-methyl-2-pyrrolidone, 2-pyrrolidone, *N, N*-dimethylformamide, *N, N*-dimethylacetamide, dimethyl sulfoxide, propylene carbonate, caprolactam, triacetin, or any combination thereof. In another embodiment, the polar aprotic solvent is *N*-methyl-2-pyrrolidone.

As set forth in U.S. Patent 6,565,875, the polar aprotic solvent can be present in any suitable amount. The type and amount of biocompatible polar aprotic solvent present in the composition may depend upon the desired properties of the controlled release implant.

In a particular embodiment, the type and amount of biocompatible polar aprotic solvent can influence the length of time in which the GnRH antagonist is released from the controlled release implant.

In one example, the flowable composition of the invention comprises a flowable delivery system such as an Atrigel^{®} system comprising a copolymer (i.e. PLGA), a water soluble organic solvent, and a GnRH antagonist (i.e. cetrorelix).

In yet another aspect, the invention relates to a method for treating a disease associated with GnRH, the method comprising administering a therapeutically effective amount of the claimed composition of the invention.

In a further aspect, the invention relates to a method of extending release of a pharmaceutical agent (i.e. cetrorelix) in a subject for a period ranging from 1 month to 6 months, the method comprising administering to said subject the claimed composition of the invention (e.g. microspheres). In another aspect, the invention relates to a method of extending the release of a pharmaceutical agent (i.e. cetrorelix) in a subject for a period of at least 90 days, the method comprising administering to said subject the claimed composition of the invention (e.g. microspheres).

In a yet further aspect, the invention relates to a method of maintaining an effective level of a therapeutic agent (i.e. cetrorelix) in a subject for a period ranging from 1 month to 6 months, the method comprising administering to said subject the claimed composition of the invention (e.g. microspheres). In another aspect, the invention relates to a method of maintaining an effective level of a therapeutic agent (i.e. cetrorelix) in a subject for a period at least 90 days, the method comprising administering to said subject the claimed composition of the invention (e.g. microspheres).

The intrinsic viscosity also may vary depending on one or more desired properties. In some embodiment, the intrinsic viscosity is larger than about 0.1 dl/g and less than about 6 dl/g. In one embodiment, the intrinsic viscosity lies between about 0.2-4 dl/g, more preferably between 0.4-2 dl/g.

In an exemplary embodiment, the composition is capable of achieving a therapeutic effect, for example, within 24 hrs and maintains therapeutic effect for at least 90 days in, for example, >95% percent of treated patients. In a particular embodiment, the composition is in the form of a hydrogel. In another particular embodiment, the composition is in the form of microspheres.

Microspheres for sustained release of therapeutically active agents and methods of their preparations are well known in the art (see e.g. U.S. Patent Nos. 6,458,387 and 9,381,159 ). The microspheres typically comprise a matrix formed of biodegradable polymer. In some embodiments, the inner matrix diffuses through the outer surface under appropriate conditions. In some embodiments, the outer surface not only allows aqueous fluids to enter the microsphere, but also allows solubilized drug and polymer to exit the microsphere. The microspheres can be made to release drug and polymer from the interior of the microsphere when placed in an appropriate aqueous medium, such as body fluids or a physiologically acceptable buffer under physiological conditions over a prolonged period of time, thereby providing sustained release of a drug. In one embodiment, the microspheres can be made to release a drug without an initial burst or rapid drug release.

The microspheres have a generally uniform size (substantially spherical) and shape, with each preparation having a narrow size distribution. Microspheres range in size from about 0.5 microns to about 100 microns, depending upon the fabrication conditions. The characteristics of the microspheres may be altered during preparation by manipulating the water soluble polymer concentration, reaction temperature, pH, concentration of therapeutic agent, crosslinking agent, and/or the length of time the macromolecule is exposed to the crosslinking agent and/or the energy source. In one example, microspheres are suitable for oral or parenteral administration; mucosal administration; ophthalmic administration; intravenous, subcutaneous, intra articular, or intramuscular injection; administration by inhalation; or topical administration.

The amount of polymer matrix (i.e. PLGA), in the microsphere composition, can be any suitable amount, known to one of skilled in the art. The amount, in the microsphere composition, may range from 10 wt. % to 50 wt. %; from about 20 wt. % to about 40 wt. %; from about 25 wt. % to about 35 wt. %. In a particular embodiment, the amount is 10, 20, 25, 30, 35, 40, 45, or 50 wt. %.

The amount of therapeutic molecule (i.e. cetrorelix) in the microsphere can range from 5 wt. %. to about 20 wt. %, from about 10 wt. %. to about 15 wt. %. In a particular embodiment, the amount is 5, 7, 9 10, 15 20, 25, 30, 35,40,45, 50, 60, 70, 80, or 90 wt. %.

The composition of the invention can be administered using a suitable method. In one aspect, the composition of the invention is an injectable composition, which is administered with one injection or two injections administered at the same time using, for example, a 21G needle or smaller, with a total injection volume, for example, less than 4 mL. Injections may be subcutaneous or intramuscular.

In another aspect, the composition of the invention allows for consistent release of the active agent from the drug delivery vehicle with no more than 25% variation plus an encapsulation efficiency of over 70%. In yet another aspect, the composition of the invention allows the releases the active agent from the drug delivery vehicle with >85% intact over the entire duration of release.

Effective doses of the compositions of the present invention, for treatment of conditions or diseases as described herein vary depending upon many different factors, including means of administration, target site, physiological state of the patient, whether the patient is human or an animal, other medications administered, and whether treatment is prophylactic or therapeutic. Usually, the patient is a human but non-human mammals including transgenic mammals can also be treated. Treatment dosages may be titrated using routine methods known to those of skill in the art to optimize safety and efficacy.

The compositions of the invention may include a "therapeutically effective amount." A "therapeutically effective amount" refers to an amount effective, at dosages and for periods of time necessary, to achieve the desired therapeutic result. A therapeutically effective amount of a molecule may vary according to factors such as the disease state, age, sex, and weight of the individual, and the ability of the molecule to elicit a desired response in the individual. A therapeutically effective amount is also one in which any toxic or detrimental effects of the molecule are outweighed by the therapeutically beneficial effects.

The disclosure further provides methods for treating a disease or condition with gonadotropin-releasing hormone (GnRH) antagonist, thereby treating said disease in said subj ect.

The compositions of the invention described herein can be used to treat any GnRH associated disease or condition that could be treated by GnRH antagonist. Examples of treatments, for diseases or conditions treated by the compositions of the invention include, for example, but not limited to, suppression of testosterone production, FSH, and LH for the treatment of prostate cancer and benign prostatic hyperplasia, directly blocking GnRH receptors on prostate cells for treatment of prostate cancer and benign prostatic hyperplasia, controlled ovarian stimulation for assisted reproductive techniques, treatment of uterine myoma, suppression of ovarian function while undergoing chemotherapy, treatment of breast cancer, treatment of ovarian cancer, male contraception, and female contraception.

As used herein, the terms "treat" and "treatment" refer to therapeutic treatment, including prophylactic or preventative measures, wherein the object is to prevent or slow down (lessen) an undesired physiological change associated with a disease or condition. Beneficial or desired clinical results include, but are not limited to, alleviation of symptoms, diminishment of the extent of a disease or condition, stabilization of a disease or condition (i.e., where the disease or condition does not worsen), delay or slowing of the progression of a disease or condition, amelioration or palliation of the disease or condition, and remission (whether partial or total) of the disease or condition, whether detectable or undetectable. "Treatment" can also mean prolonging survival as compared to expected survival if not receiving treatment. Those in need of treatment include those already with the disease or condition as well as those prone to having the disease or condition or those in which the disease or condition is to be prevented.

"Administration" to a subject is not limited to any particular delivery system and may include, without limitation, parenteral (including subcutaneous, intravenous, intramedullary, intraarticular, intramuscular, or intraperitoneal injection).

The composition of the invention may be administered parenterally (e.g., intravenous, subcutaneous, intraperitoneal, and intramuscular). Further, the composition of the invention may be administered by intravenous infusion or injection. The composition of the invention may be administered by intramuscular or subcutaneous injection. In some embodiments, the composition of the invention may be administered surgically. As used herein, a "composition" refers to any composition that contains a pharmaceutically effective amount of one or more active ingredients (e.g., a GnRH antagonist).

The methods of treatment described herein can be used to treat any suitable mammal, including primates, such as monkeys and humans, horses, cows, cats, dogs, rabbits, elk, deer and rodents such as rats and mice. In one embodiment, the mammal to be treated is human..

### EXAMPLES

Compositions which do not contain cetrorelix are not covered by the claimed invention.

### EXAMPLE 1

Poly(DL-lactide-co-glycolide) with 50:50 ratio of lactide to glycolide can be dissolved in a suitable solvent to prepare an Atrigel^{®} polymer solution. This solution can be filled into a syringe with a female luer lock fitting.

Each GnRH antagonist (ozarelix, degarelix, cetrorelix, or ganirelex) can be dissolved in water or other solvents and filled into a syringe with a male luer-lock fitting.

Prior to administration, the two syringes can be coupled and the contents can be mixed back and forth between the two syringes for multiple cycles. After thorough mixing, the formulation can be drawn back into the syringe with the male coupling.

Then, the two syringes can be separated and a needle (a 21G needle or smaller) can be attached. The contents of the syringe can then be subcutaneously injected into subjects. A total injection volume can be less than 4 mL.

Serum can be collected and analyzed. The GnRH antagonist composition may achieve a therapeutic effect within 24 hrs and maintain therapeutic effect for at least 90 days in >95% percent of treated patients.

The composition may allow for consistent release of the active agent from the drug delivery vehicle with no more than 25% variation plus an encapsulation efficiency of over 70%. The composition may release the active agent from the drug delivery vehicle with >85% intact over the entire duration of release.

### EXAMPLE 2

A multi-block copolymer is provided. Each GnRH antagonist (ozarelix, degarelix, cetrorelix, or ganirelex) can be loaded into the multi-block copolymer. The formulation may be in the form of microspheres.

A syringe with a 21G needle or smaller can be used to inject the formulation. The formulation can be subcutaneously injected into subjects. A total injection volume can be less than 4mL.

Serum can be collected and analyzed. The GnRH antagonist composition may achieve a therapeutic effect within 24 hrs and maintain therapeutic effect for at least 90 days in >95% percent of treated patients.

The composition may allow for consistent release of the active agent from the drug delivery vehicle with no more than 25% variation plus an encapsulation efficiency of over 70%. The composition may release the active agent from the drug delivery vehicle with >85% intact over the entire duration of release.

### EXAMPLE 3

### Development of Cetrorelix Microspheres Formulations

Several formulations of microspheres using different polymers and internal water phase compositions were prepared for testing cetrorelix *in vitro* release (IVR). The tested formulations are summarized in **Table 1**

**Table 1 Initial Cetrorelix Formulations**

| **MSP Batch** | **Process** | **Polymer** | **Microsphere morphology** | **Microsphere size (D50) (µm)** | **Theoretical CRX loading (Wt.%)** | **CRX Loading measured by EAS (Wt.%)** | **EE (%)** |
|---|---|---|---|---|---|---|---|
| **AD17-008** | W1/O/W2 (W1= Acetic acid/H₂O 50/50) | 10CP10C20-D23 | Spherical, monodispersed | 40 | 12.5 | 11 | 88.5 |
| **RP17-004** | W1/O/W2 (W1 = Acetic acid/H₂O 35/65 pre-mix) | 10CP10C20-D23 | Spherical, monodispersed | 73 | 14.3 | 13.8 | 96.5 |
| **RP17-006** | W1/O/W2 (W1= Acetic acid/H₂O 35/65 pre-mix) | 10LP10L20-LL40 | Spherical, monodispersed | 71 | 14.0% | 14.8 | 105.4 |

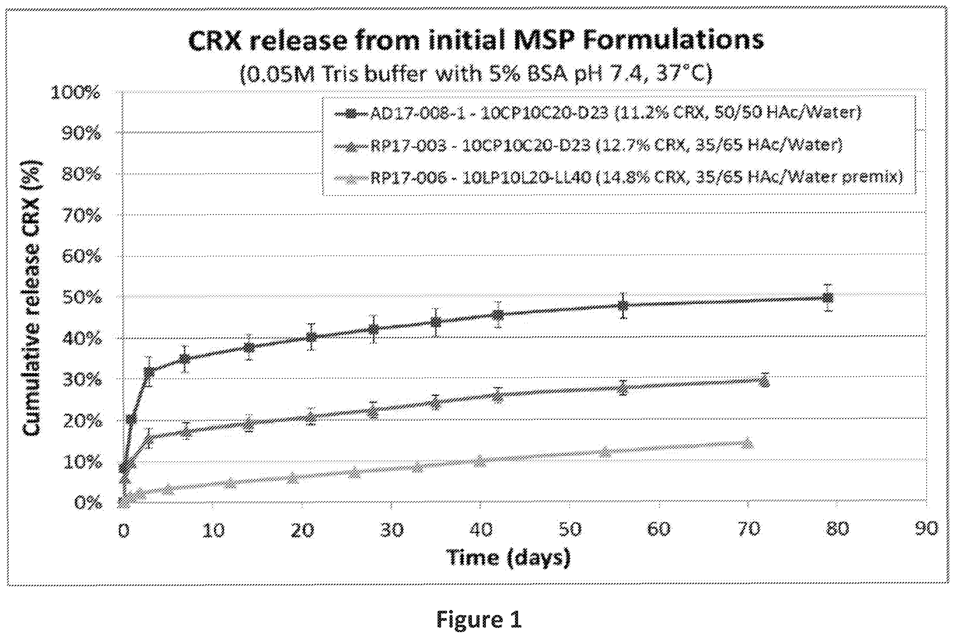

The *in vitro* release of cetrorelix was tested by incubating microsphere formulations listed in **Table 1** in 0.05 M Tris Buffer with 5% BSA, pH 7.4 at 37°C. The results show the release was slowest when premixed 35% Acetic acid/ 65% H₂O as internal water phase was used (**Figure 1**).

To further test cetrorelix IVR several formulations of cetrorelix-loaded microspheres using different polymers and 35% Acetic acid/ 65% H₂O as internal water phase were made **(Table 2).**

**Table 2 Cetrorelix Formulations manufactured with optimizing primary emulsification process (W1 = 36/65 Acetic acid/ Water mixture)**

| **MSP Batch** | **Process** | **Polymer** | **Microsphere morphology** | **MSP size (D50) (µm)** | **Theoretical CRX loading (Wt.%)** | **CRX Loading measured by EAS (Wt.%)** | **EE (%)** |
|---|---|---|---|---|---|---|---|
| **RP17-012** | W1/O/W2 | 10CP10C20-D23 | Spherical, monodisperse d | 82 | 15.70% | 13.5 | 85.70% |
| **RP17-013** | W1/O/W2 | 20CP15C50-D23 | Spherical, monodisperse d | 85 | 14.00% | 13.4 | 95.70% |
| **RP17-014** | W1/O/W2 | 20LP10C20-LL40 | Spherical, monodisperse d | 70 | 13.90% | 13.6 | 97.80% |
| **RP17-015** | W1/O/W2 | 20CP10C20-LL40 | Spherical, monodisperse d | 56 | 13.20% | 11.9 | 90.90% |
| **RP17-018** | W1/O/W2 | 30CP15C50-D23 | Spherical, monodisperse d | 51 | 13.80% | 9.5 | 68.40% |

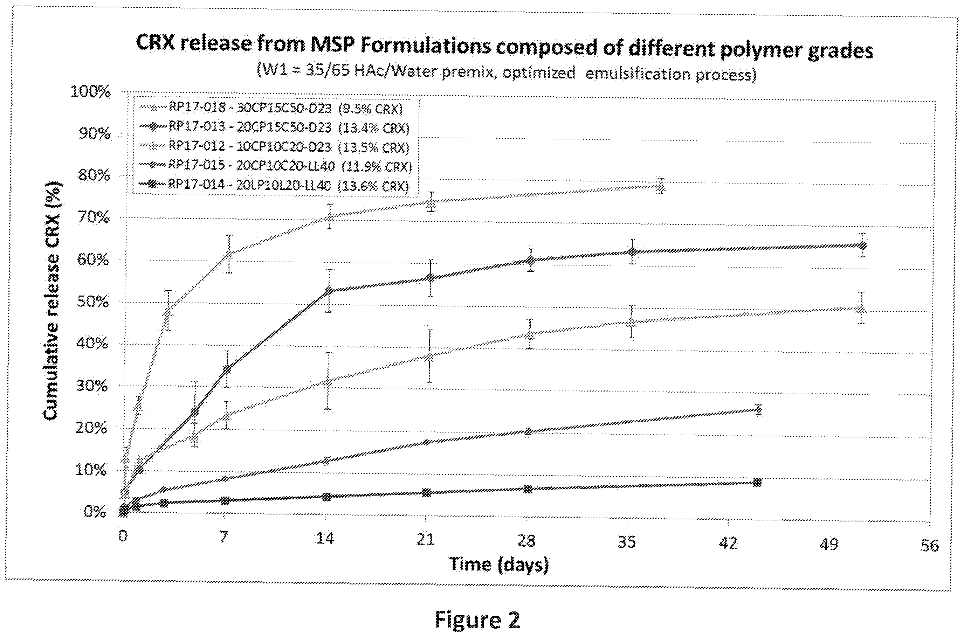

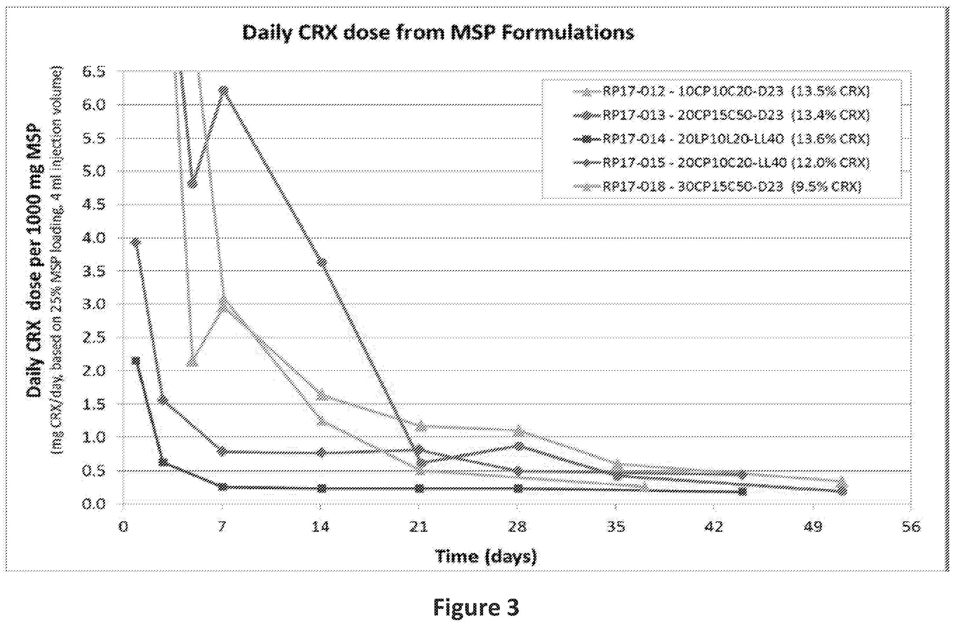

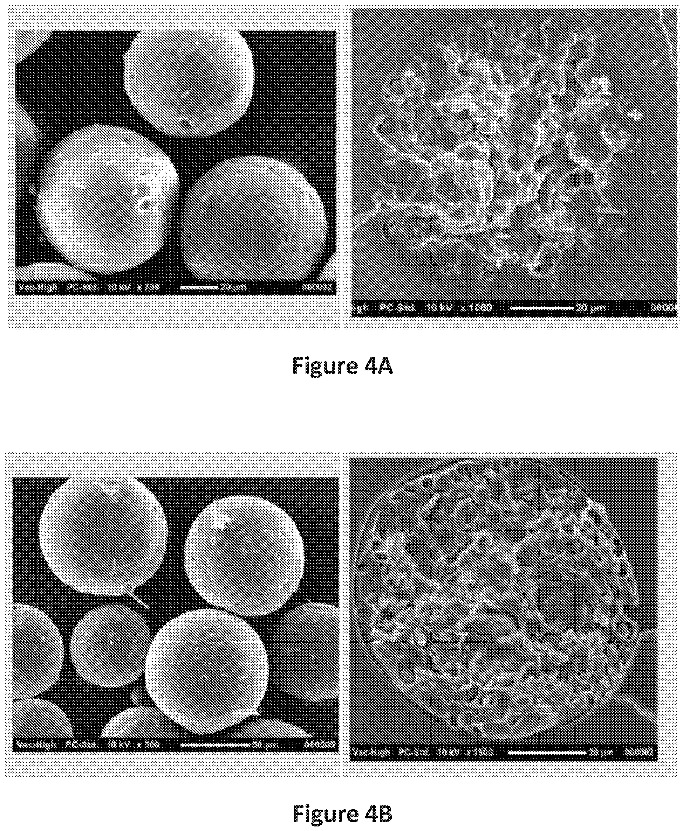

The *in vitro* release of cetrorelix was tested by incubating microsphere formulation listed in **Table 2** in 0.05 M Tris Buffer with 5% BSA, pH 7.4 at 37°C. The results show that the RP17-014 formulation using 20LP10C20-LL40 polymer had the slowest cetrorelix release rate, and, in addition shown linear release kinetics **(****Figure 2****),** while RP17-012 (10CP10C20-D23) and RP17-012 (10CP10C20-D23) microsphere formulations (depicted in electron micrographs in **Figure 4A** and **Figure 4B** respectively) provided the highest sustained daily dose of cetrorelix **(****Figure 3****).**

These results show that slow degrading L-Lactide based polymers with low swellability are suitable for use in cetrorelix-loaded microspheres and display linear cetrorelix release. In addition the microsphere manufacturing process achieves cetrorelix encapsulation of up to 15%.

### EXAMPLE 4

### Cetrorelix-Loaded Microspheres Pharmacokinetics in Rats

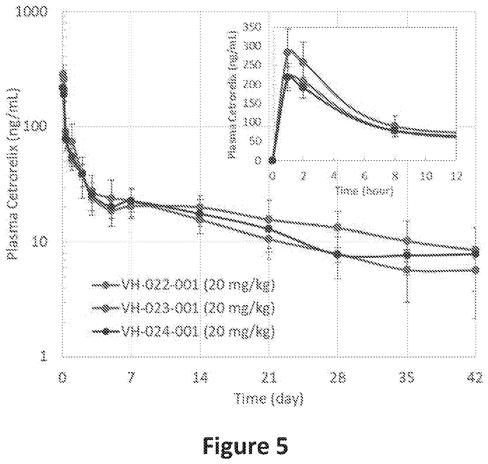

Several salt-free cetrorelix-loaded microsphere formulations having different polymer contents were used for PK Study **(Table3).** The preparations (<1 ml) were subcutaneously implanted into rats at a single 20 mg/kg dose and cetrorelix levels in plasma were monitored over 6 weeks. The results are summarized in **Table 4** and **Figure 5****.** All formulations showed detectable plasma cetrorelix ≥ 42 days. Cₘₐₓ/C_{ave,42day} varied from 12.1 to 17.6 and the percentage of day 1 release related to 42 days ranged from 12 to 17%. Importantly, the amount of polymer in the preparation did not seem to have any statistically significant effect on cetrorelix release.

**Table 3 Salt-free Cetrorelix-loaded microsphere formulations**

| **ID** | **Cetroreli x(mg)** | **RG502H (mg)** | **RG752H (mg)** | **NMP (mg)** | **Total (mg)** | **Theo. Cetroreli x Loading (%,w/w)** | **PLGA Solution (%,w/w)^{∗}** | **Total Solid (%,w/w)** |
|---|---|---|---|---|---|---|---|---|
| **VH-022-001** | 365.5 | 677.22 | 0.0 | 2688 | 3730.4 | 9.8 | 20.1% | 28.0 |
| **VH-023-001** | 291.3 | 408.28 | 405.7 | 1885 | 2990.5 | 9.7 | 30.2% | 37.0 |
| **VH-024-001** | 291.5 | 0.0 | 1084.6 | 1618 | 2993.6 | 9.7 | 40.1% | 46.0 |

| | | | | | | | | |
|---|---|---|---|---|---|---|---|---|
| ^{∗} PLGA (%,w/w) = PLGA wt + (PLGA wt + Total Solvent wt) | | | | | | | | |

**Table 4 Salt-free Cetrorelix-loaded microspheres Rat pharmacokinetics results**

| **ID** | **Cₘₐₓ (ng/mL)** | **Tₘₐₓ (hr)** | **AUC_{0-42day} (ng/mL ^{∗} hr)** | **C_{ave,42doy} (ng/mL)** | **Cₘₐₓ/C_{ave, 42day}** | **AUC₀₋₂₄ₕᵣ (ng/mL ^{∗} hr)** | **AUC₀₋₂₄ₕᵣ/AUC₀₋ 42day** |
|---|---|---|---|---|---|---|---|
| **VH-022-001** | 283.7 | 1 | 16272.7 | 16.1 | 17.6 | 2761.9 | 17.0 % |
| **VH-023-001** | 218.7 | 1 | 18270.5 | 18.1 | 12.1 | 2206.9 | 12.1% |
| **VH-024-001** | 218.7 | 1 | 16406.1 | 16.3 | 13.4 | 2191.0 | 13.4% |

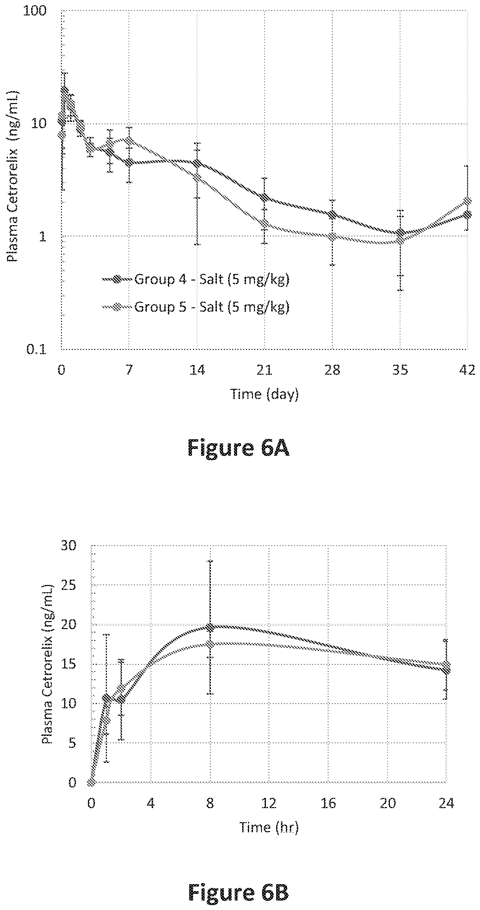

In addition, pharmacokinetics of cetrorelix microspheres formulations containing either Ca Pamoate or Na Oleate salt were tested in rats through subcutaneously implanting a single 5 mg/kg microspheres dose in a vehicle solution (20 mM K-Phos Buffer, 2.5% Mannitol, 3.5% CMC, 0.1% PS80) and monitoring cetrorelix levels in plasma over 6 weeks. The results are summarized in **Table 5** and **Figures 6A** and **6B****.** All formulations showed detectable plasma cetrorelix ≥ 42 days. Cₘₐₓ/C_{ave,42day} varied from 2.4 to 3.1 and the percentage of day 1 release related to 42 days about 11%.

**Table 5 Salt-containing Cetrorelix formulations Rat pharmacokinetics results.**

| **ID** | **Cₘₐₓ (ng/mL)** | **Tₘₐₓ (hr)** | **AUC _{0-42day} (ng/mL ^{∗} hr)** | **C ave, 42day (ng/mL)** | **Cₘₐₓ/ C_{ave,42day}** | **AUC 0-24hr (ng/mL ^{∗} hr)** | **AUC₀₋₂₄ₕᵣ**/ **AUC_{0-42day}** |
|---|---|---|---|---|---|---|---|
| **Group 4** - **Salt Ca Pamoate** | 19.6 | 8 | 3444.0 | 3.4 | 3.1 | 376.9 | 10.9% |
| **Group 5** - **Salt Na Oleate** | 17.5 | 8 | 3331.9 | 3.3 | 2.4 | 361.1 | 10.8% |

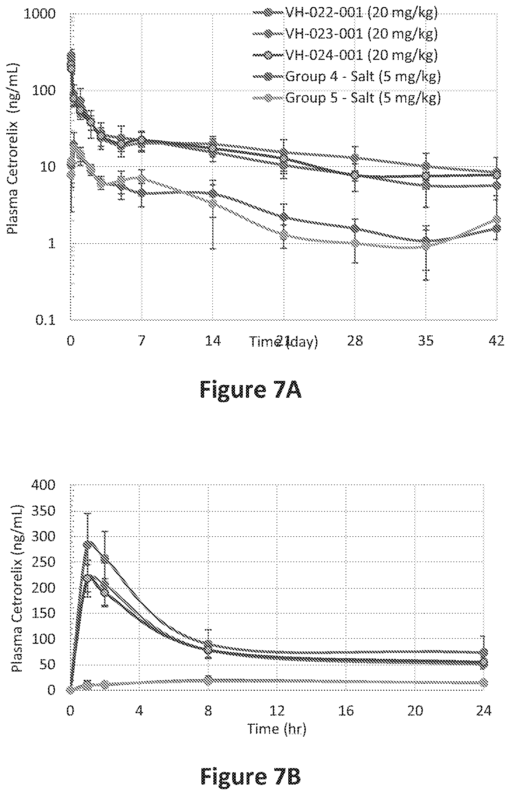

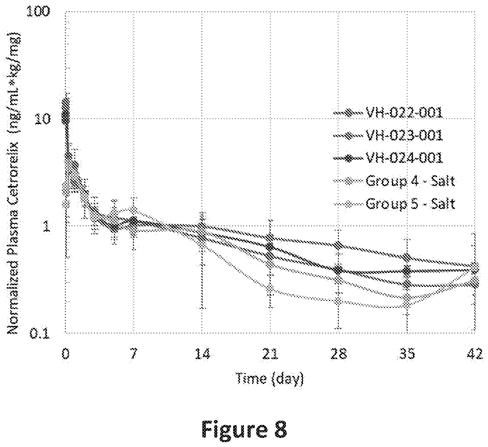

There was no principal difference between pharmacokinetics of cetrorelix microspheres formulations containing Ca Pamoate and those containing Na Oleate both in the initial 24 hour burst release **(****Figure 6B****)** and over the long term **(****Figure 6A****).** Moreover, while salt-containing cetrorelix microspheres resulted in lower plasma cetrorelix compared to the salt free formulations in the long term **(****Figure 7A****),** this difference could be attributable to the different dose **(****Figure 8****).** However, dose difference did not account for the far greater peak concentration observed at the initial burst release phase when salt free formulations were used. Indeed, addition of salt all but eliminated the sharp peak observed in its absence **(****Figure 7b****)**

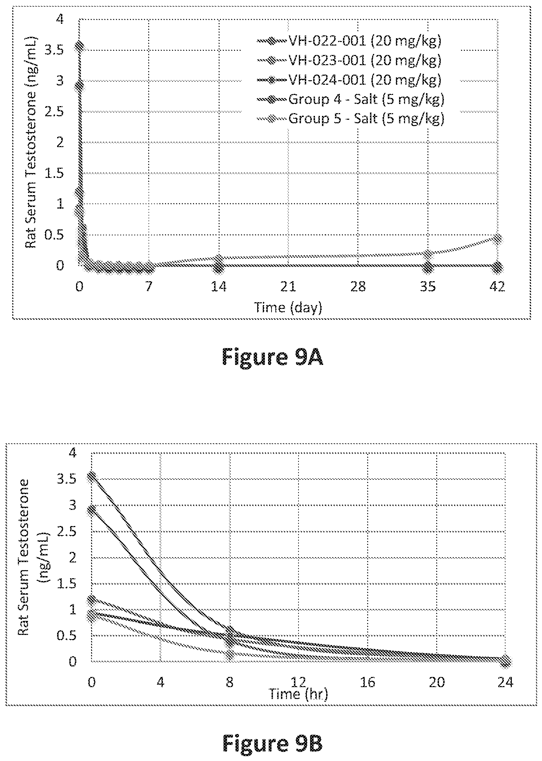

The downstream physiological effects of cetrorelix microspheres administration were assessed through monitoring testosterone levels in rats. All the formulations caused notable drop in serum testosterone levels after 24 hours **(****Figure 9B****).** These low levels were maintained for the entire 6 weeks monitoring period in all rats except those treated with Na Oleate- containing microspheres **(****Figure 9A****).**

The total amount of cetrorelix released was assessed using the method of Schwahn et al., Drug Metabolism & Disposition, Vol. 28, No. 1, p10, assuming rat weight ranging from 250 to 400 g, 10 mg/kg dose, and AUC (ng/mL ^{∗}hr) = 618.1.

Based on these assumptions, AUC for 20 mg/kg Dose (ng/mL ^{∗}hr) was assumed to be 123,620 and AUC for 5 mg/kg Dose (ng/mL ^{∗}hr) was assumed to be 30,905. The calculations based on the above assumptions results in estimated percentage of cetrorelix released (up to 42 days) ranging from 11 to 15 % of the amount initially present in the microspheres. (See **Table 6)**

**Table 6 Summary of pharmacokinetic data for Cetrorelix microspheres formulation**

| **ID** | **PLGA or Salt** | **Dose (mg/kg)** | **Cₘₐₓ (ng/mL)** | **Tₘₐₓ (hr)** | **AUC_{0-42day} (ng/mL ∗ hr)** | **Est. % Cetrorelix Released (up to 42 days)** |
|---|---|---|---|---|---|---|
| **VH-022-001** | RG502H, 20% | 20 | 283.7 | I | 16272.7 | 13.2 |
| **VH-023-001** | RG502H/RG752H, 30% | 20 | 218.7 | I | 18270.5 | 14.8 |
| **VH-024-001** | RG752H, 40% | 20 | 218.7 | I | 16406.1 | 13.3 |
| **Group 4** - **Salt** | Ca Pamoate | 5 | 19.6 | 8 | 3444.0 | 11.1 |
| **Group 5** - **Salt** | Na Oleate | 5 | 17.5 | 8 | 3331.9 | 10.8 |

### EXAMPLE 5

### In Vitro Cetrorelix Release Studies of Additional Microsphere Formulations

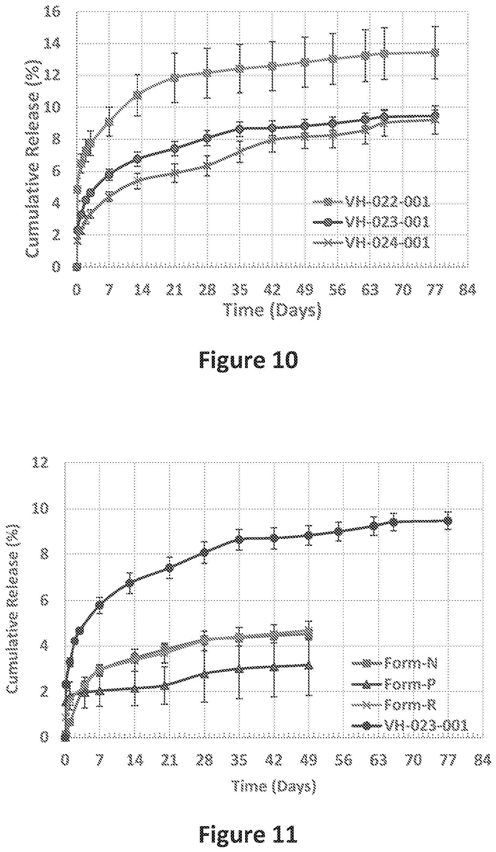

In order to supplement the *in vivo* pharmacokinetic data and further optimize the compositions of cetrorelix-loaded microspheres *in vitro* release studies were carried out wherein 45 mg of microspheres were incubated in 0.5 ml Tris Mannitol, pH 7.4 at 37°C. The results for *in vitro* release for the salt-free formulations used in pharmacokinetic studies are summarized in **Figure 10****.** In contrast to the *in vivo* data, the increase of PLGA content in the composition from 20% to 30% resulted in marked reduction of cetrorelix release rate and of cumulative release. Surprisingly, further increase of PLGA content from 30% to 40%, while resulting in further decrease in release rate, also yielded negligible change in cumulative release levels, especially in the long term. Moreover in all formulations the cumulative release appears to plateau after about 60 days suggesting that the maximum cumulative release has been achieved.

In order to further explore the effect of salt and polymer on cetrorelix release, several formulations of salt-containing 15% RG502H (50:50 lactide:glycolide) and 15% RG752H (75:25 lactide:glycolide) (30% total PLGA content) cetrorelix-loaded microspheres (Table 7) were tested and compared with salt-free formulations.

**Table 7 Salt-containing RG502H/RG752H (30% PLGA) cetrorelix-loaded microsphere formulations**

| **ID** | **Salt** | **Cetrorelix Salt (mg)** | **Cetrorelix Content (%,w/w)** | **PLGA^{∗} (mg)** | **NMP (mg)** | **Total (mg)** | **Cetrorelix Loading (%,w/w)** | **PLGA Solution (%,w/w)** | **Total Solid (%,w/w)** |
|---|---|---|---|---|---|---|---|---|---|
| **Form-N** | Ca Pamoate | 63.29 | 77% | 135.0 | 316.3 | 514.6 | 9.5 | 29.9 | 38.5 |
| **Form-P** | Na Oleate | 58.80 | 83% | 134.7 | 317.4 | 510.9 | 9.6 | 29.8 | 37.9 |
| **Form-R** | Ca Citrate | 62.20 | 78% | 134.9 | 316.5 | 513.6 | 9.4 | 29.9 | 38.4 |

| | | | | | | | | | |
|---|---|---|---|---|---|---|---|---|---|
| ^{∗} PLGA = 50:50 RG752H:RG502H (w:w) | | | | | | | | | |

The results show that the presence of salt markedly decreases cumulative cetrorelix release levels. While Na Oleate was particularly effective **(****Figure 11****),** all salts resulted in lower cumulative release compared to corresponding salt free concentration. In addition, cetrorelix cumulative release appeared to reach plateau after 49 days. At the same time Na Oleate and Ca Citrate did not appear to have any effect on the initial release rate while it was slightly decreased in the presence of Ca Pamoate.

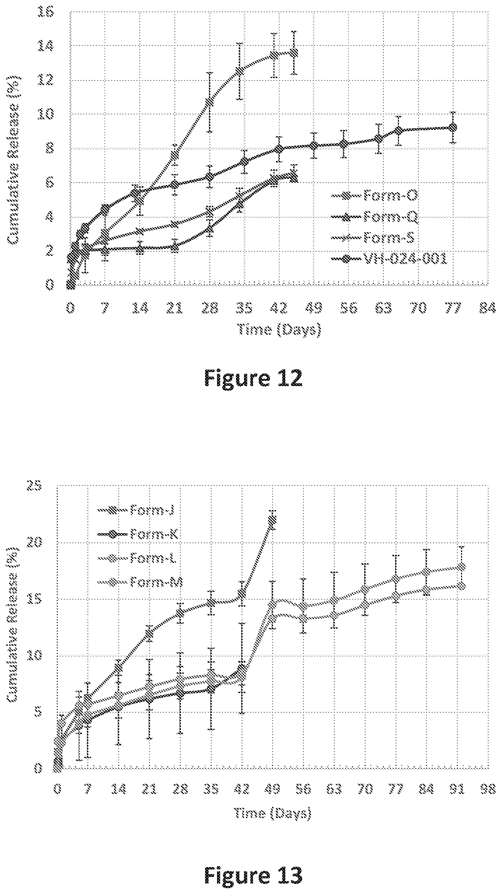

Surprisingly, when the polymer content was increased to 40% and replaced with RG752H (75:25 lactide:glycolide) **(Table 8)** the presence of Ca Pamoate slowed the initial release rate but dramatically (nearly 3-fold) increased cumulative cetrorelix release, as compared with salt free formulation. On the other hand, similarly to RG502H/RG752H data, Na Oleate and Ca Citrate did not appear to have any effect on the initial release rate although these formulations displayed a modest increase in cumulative release levels **(****Figure 12****).**

**Table 8 Salt-containing RG752H (40% PLGA) Cetrorelix-loaded microsphere formulations**

| **ID** | **Salt** | **Cetrorelix Salt (mg)** | **Cetrorelix Content (%,w/w)** | **RG752H (mg)** | **NMP (mg)** | **Total (mg)** | **Cetrorelix Loading (%,w/w)** | **PLGA Solution (%,w/w)** | **Total Solid (%,w/w)** |
|---|---|---|---|---|---|---|---|---|---|
| **Form-O** | Ca Pamoate | 62.95 | 77% | 180.5 | 268.4 | 511.8 | 9.5 | 40.2 | 47.6 |
| **Form-Q** | Na Oleate | 58.53 | 83% | 180.6 | 269.4 | 508.6 | 9.6 | 40.1 | 47.0 |
| **Form-S** | Ca Citrate | 56.88 | 78% | 166.2 | 246.5 | 469.5 | 9.4 | 40.3 | 47.5 |

In an attempt to investigate the effect of polymer and N-methyl-2-pyrrolidone (NMP) on cetrorelix release rate and cumulative release, microsphere formulations using 20% RG502H and 20% RG752H, or 40% RG752H, with and without NMP **(Table 9)** were tested.

**Table 9 RG502H/RG752H (40% PLGA) Cetrorelix-loaded microsphere formulations with and without NMP.**

| **ID** | **Cetrorelix (mg)** | **RG752S (mg)** | **RG502H + RG752H (mg)*** | **NMP (mg)** | **DMSO (mg)** | **Total (mg)** | **Cetrorelix Loading (%,w/w)** | **PLGA Solution (%,w/w)**** | **Total Solid (%,w/wl** |
|---|---|---|---|---|---|---|---|---|---|
| Form-J | 45.3 | 167.43 | 0 | 257 | 0 | 470.1 | 9.6 | 39.4 | 45.3 |
| Form-K | 45.4 | 168.55 | 0 | 0 | 251 | 465.2 | 9.8 | 40.1 | 46.0 |
| Form-L | 45.6 | 0 | 166.98 | 252 | 0 | 464.2 | 9.8 | 39.9 | 45.8 |
| Form-M | 45.9 | 0 | 169.87 | 0 | 252 | 467.3 | 9.8 | 40.3 | 46.2 |

| | | | | | | | | | |
|---|---|---|---|---|---|---|---|---|---|
| ^{∗} RG502H:RG752H (w:w) = 50:50 ^{∗∗} PLGA (%,w/w) = PLGA wt + (PLGA wt + Total Solvent wt) | | | | | | | | | |

The results are summarized in **Figure 13** and **Table 10.** Surprisingly, omitting NMP from 40%RG752H resulted in dramatic decrease in both cetrorelix release rate and total release. Interestingly, 20% RG502H / 20% RG752H microspheres displayed NMP-independent multiphasic cetrorelix release profile consisting of initial burst phase followed by a first plateau phase at about 8% cumulative release, achieved on day 42, followed by the second burst phase on days 42-49, and subsequently followed by a second plateau phase, at approximately 17% cumulative release, achieved on day 92.

**Table 10 In vitro Cetrorelix release by 40% RG752H - microsphere formulations with and without NMP**

| **ID** | **Total Release (%)** | **Residual Loading (%)** | **Total Recovery (%)** |
|---|---|---|---|
| **Form-J** | 22.0 (743.3 µg) | 89.0 | 111.0 |
| **Form-K** | 11.1 (362.0 µg) | 89.4 | 100.2 |

## Claims

1. A composition comprising cetrorelix and a polymer, said polymer comprising a poly-lactic co-glycolic acid (PLGA) in a lactide : glycolide molar ratio between 50:50 and 100:0, wherein cetrorelix is present in an amount of 5% - 90% of the mass of said composition, and said polymer is present in an amount of 10% - 50% of the mass of said composition, and wherein said composition is capable of extending the release of cetrorelix in a subject for a period ranging from 1 month to 6 months.

2. The composition of claim 1, wherein said composition is capable of releasing cetrorelix for more than 90 days.

3. The composition of claim 1, wherein said composition is in the form of a hydrogel.

4. The composition of claim 1, wherein said composition is a flowable composition.

5. The composition of claim 1, wherein said composition is in the form of an implant.

6. The composition of claim 1, wherein the lactide : glycolide weight ratio of said PLGA is 55:45, 60:40, 65:35, 70:30, 75:25, 80:20, 85:15, 90:10, or 95:5.

7. The composition of claim 1 further comprising a solvent.

8. The composition of claim 7, wherein said solvent is a polar aprotic solvent.

9. The composition of claim 8, wherein said solvent is *N*-methyl-2-pyrrolidone.

10. The composition of claim 7, wherein said solvent is present at the concentration ranging from 10% to 30% (w/w).

11. The composition of claim 7, wherein said PLGA comprises equal parts lactide and glycolide.

12. The composition of claim 7, wherein said PLGA comprises 75% lactide and 25% glycolide.

13. The composition of claim 7, wherein said PLGA comprises equal parts of a first and a second polymer composition, wherein said first polymer composition comprises equal parts lactide and glycolide and said second polymer composition comprises 75% lactide and 25% glycolide.

14. The composition of claim 13, wherein polymer is present at the concentration ranging from 20% to 40% (w/w).

15. The composition of claim 7 further comprising a salt; preferably wherein said salt is Ca pamoate, Na oleate, or Ca citrate.

## Patentansprüche

1. Eine Zusammensetzung, die Cetrorelix und ein Polymer umfasst,
wobei das Polymer ein Poly(lactid-co-glycolid) (PLGA) in einem Molverhältnis von Lactid:Glycolid zwischen 50:50 und 100:0 umfasst,
wobei Cetrorelix in einer Menge von 5 % - 90 % der Masse der Zusammensetzung vorhanden ist und das Polymer in einer Menge von 10 % - 50 % der Masse der Zusammensetzung vorhanden ist, und
wobei die Zusammensetzung in der Lage ist, die Freisetzung von Cetrorelix in einem Subjekt für einen Zeitraum von 1 Monat bis 6 Monaten zu verlängern.

2. Die Zusammensetzung gemäß Anspruch 1, wobei die Zusammensetzung in der Lage ist, Cetrorelix für mehr als 90 Tage freizusetzen.

3. Die Zusammensetzung gemäß Anspruch 1, wobei die Zusammensetzung in Form eines Hydrogels vorliegt.

4. Die Zusammensetzung gemäß Anspruch 1, wobei die Zusammensetzung eine fließfähige Zusammensetzung ist.

5. Die Zusammensetzung gemäß Anspruch 1, wobei die Zusammensetzung in Form eines Implantats vorliegt.

6. Die Zusammensetzung gemäß Anspruch 1, wobei das Lactid:Glycolid-Gewichtsverhältnis des PLGA 55:45, 60:40, 65:35, 70:30, 75:25, 80:20, 85:15, 90:10 oder 95:5 ist.

7. Die Zusammensetzung gemäß Anspruch 1, wobei die Zusammensetzung weiterhin ein Lösungsmittel umfasst.

8. Die Zusammensetzung gemäß Anspruch 7, wobei das Lösungsmittel ein polares aprotisches Lösungsmittel ist.

9. Die Zusammensetzung gemäß Anspruch 8, wobei das Lösungsmittel N-Methyl-2-pyrrolidon ist.

10. Die Zusammensetzung gemäß Anspruch 7, wobei das Lösungsmittel in einer Konzentration im Bereich von 10 Gew.-% bis 30 Gew.-% vorliegt.

11. Die Zusammensetzung gemäß Anspruch 7, wobei das PLGA gleiche Teile Lactid und Glycolid umfasst.

12. Die Zusammensetzung gemäß Anspruch 7, wobei das PLGA 75 % Lactid und 25 % Glycolid umfasst.

13. Die Zusammensetzung gemäß Anspruch 7, wobei das PLGA gleiche Teile einer ersten Polymerzusammensetzung und einer zweiten Polymerzusammensetzung umfasst, wobei die erste Polymerzusammensetzung gleiche Teile Lactid und Glycolid umfasst und die zweite Polymerzusammensetzung 75 % Lactid und 25 % Glycolid umfasst.

14. Die Zusammensetzung gemäß Anspruch 13, wobei das Polymer in einer Konzentration von 20 Gew.-% bis 40 Gew.-% vorliegt.

15. Die Zusammensetzung gemäß Anspruch 7, wobei die Zusammensetzung weiterhin ein Salz umfasst, wobei das Salz bevorzugt Ca-Pamoat, Na-Oleat oder Ca-Citrat ist.

## Revendications

1. Composition comprenant du cétrorélix et un polymère, ledit polymère comprenant un poly(acide lactique-co-glycolique) (PLGA) en un rapport molaire de lactide : glycolide entre 50 : 50 et 100 : 0, le cétrorélix étant présent en une quantité de 5 % à 90 % de la masse de ladite composition, et ledit polymère étant présent en une quantité de 10 % à 50 % de la masse de ladite composition, et ladite composition étant capable de prolonger la libération de cétrorélix chez un sujet pendant une période dans la plage de 1 mois à 6 mois.

2. Composition selon la revendication 1, ladite composition étant capable de libérer le cétrorélix pendant plus de 90 jours.

3. Composition selon la revendication 1, ladite composition étant sous la forme d'un hydrogel.

4. Composition selon la revendication 1, ladite composition étant une composition fluide.

5. Composition selon la revendication 1, ladite composition étant sous la forme d'un implant.

6. Composition selon la revendication 1, le rapport en poids de lactide : glycolide dudit PLGA étant de 55 : 45, 60 : 40, 65 : 35, 70 : 30, 75 : 25, 80 : 20, 85 : 15, 90 : 10 ou 95 : 5.

7. Composition selon la revendication 1 comprenant en outre un solvant.

8. Composition selon la revendication 7, ledit solvant étant un solvant aprotique polaire.

9. Composition selon la revendication 8, ledit solvant étant la *N*-méthyl-2-pyrrolidone.

10. Composition selon la revendication 7, ledit solvant étant présent à la concentration dans la plage de 10 % à 30 % (p/p).

11. Composition selon la revendication 7, ledit PLGA comprenant des parties égales de lactide et de glycolide.

12. Composition selon la revendication 7, ledit PLGA comprenant 75 % de lactide et 25 % de glycolide.

13. Composition selon la revendication 7, ledit PLGA comprenant des parties égales d'une première et d'une deuxième composition de polymère, ladite première composition de polymère comprenant des parties égales de lactide et de glycolide et ladite deuxième composition de polymère comprenant 75 % de lactide et 25 % de glycolide.

14. Composition selon la revendication 13, le polymère étant présent à la concentration dans la plage de 20 % à 40 % (p/p).

15. Composition selon la revendication 7 comprenant en outre un sel ; préférablement ledit sel étant le pamoate de Ca, l'oléate de Na ou le citrate de Ca.
